Europäisches Patentamt

European Patent Office

Office européen des brevets

(19)

(11) Publication number: **0 135 129**
**A2**

(12) # EUROPEAN PATENT APPLICATION

(21) Application number: **84109597.9**

(22) Date of filing: **11.08.84**

(51) Int. Cl.⁴: **G 01 N 33/531**
**G 01 N 33/545**
**//B01J13/02**

(30) Priority: **12.08.83 JP 147366/83**

(43) Date of publication of application:
**27.03.85 Bulletin 85/13**

(84) Designated Contracting States:
**DE GB**

(71) Applicant: **FUJI PHOTO FILM CO., LTD.**
**210 Nakanuma Minami Ashigara-shi**
**Kanagawa 250-01(JP)**

(72) Inventor: **Akiyoshi, Yutaka**
**3-11-46, Senzui**
**Asaka-shi Saitama(JP)**

(74) Representative: **Werner, Hans-Karsten, Dr. et al,**
**Deichmannhaus am Hauptbahnhof**
**D-5000 Köln 1(DE)**

(54) **Reagent for immunological determination.**

(57) A reagent for immunological determination in the form of a carrier such as a microparticle (e.g. microcapsule) or a sheet-type carrier which is provided on the surface with poly(vinyl alcohol) having a saponification degree of not higher than 90% and sensitized with an immunologically active component such as antigen, antibody, or an enzyme-labeled immunologically active component.

EP 0 135 129 A2

0135129

## REAGENT FOR IMMUNOLOGICAL DETERMINATION

## BACKGROUND OF THE INVENTION

### Field of the invention

This invention relates to a reagent for immunological determination.  More particularly, this invention relates to a reagent for determination of immunological reaction which has excellent functions such as high S/N ratio and is in the form of a microparticle or a sheet-type carrier.

### Description of prior arts

There is known a reagent for immunological determination based on immunological reaction such as antigen-antibody reaction or immunological reaction utilizing enzyme-labeled immunologically active component.  For determination of an antigen-antibody reaction with high sensitivity in a simple manner, an immunological agglutination method comprising visual observation on agglutination caused by antigen-antibody reaction utilizing antigen or antibody carried on a water-insoluble particulate carrier has been employed.

As the carriers for the immunological agglutination method, red blood cells of animals such as chicken, alligator, sheep, etc., have been used.  Using one of these red blood cell carriers, passive heamagglutination (PHA) has generally been carried out for the immunological determination.  This method provides high sensitivity with simple operations.

Recently, a method for semi-quantitatively determining antigen or antibody very efficiently in a simple manner (called "microtiter system") has ben developed. However, the microtiter system involves disadvantageous problems, for instance, caused by the use of animal-originating carriers. The red cell carriers per se are antigenic and often cause specific agglutination to adversely affect the desired antigen-antibody reaction. Further, efficiency is not uniform depending on variation of the material under analysis, change of its characteristics with the passage of time, etc., and the cost involved is rather high.

A latex agglutination method using polystyrene latex as the carrier has also been employed. Although this method is free from the disadvantageous feature encountered in the use of animal-originating carrier, this method has other disadvantages, such as poor sensitivity as compared to the passive haemagglutination method, and poor preservability due to weak bonding to the antigen or antibody. Further, natural agglutination, that is, agglutination independent of antigen-antibody reaction tends to occur in this method.

In UK Patents 2,041,517B and 2,079,936B, use of a microcapsule in place of the conventional carriers is disclosed. According to these disclosures, in the case that the microcapsule is employed as the carrier, a marked improvement in the sensitivity and accuracy is accomplished in the determination of various antibodies or antigens. This is because that microcaplules having a desired specific gravity of 0.8 to 1.20 g/cm$^3$, preferably 1.07 to 1.16 g/cm$^3$, can be easily prepared in a desired particle size range of 0.1 to 30 μm, preferably 0.5 to 10 μm, by suitably choosing the core substance of the microcapsules.

## SUMMARY OF THE INVENTION

A primary object of the present invention is to provide a reagent for immunological determination which is particularly improved in the sensitivity or S/N ratio.

A secondary object of the invention is to provide a reagent for immunological determination which is improved in the sensitivity or S/N ratio, as well as free from disadvantageous features attached to the conventional reagent of the same purpose.

There is provided by the present invention a reagent for immunological determination in the form of a carrier which is provided on the surface with poly(vinyl alcohol) having a saponification degree of not higher than 90% and sensitized with an immunologically active component.

In the present invention, a preferred carrier is a microcapsule.

## DETAILED DESCRIPTION OF THE INVENTION

It has been known that PVA (i.e. poly(vinyl alcohol) can be used for the preparation of microcapsules for inclusion in a pressure sensitive paper (Japanese Patent Provisional Publication No. 55(1980)-132631). For the microcapsule employed in a pressure sensitive paper, the density of a capsule wall is one of the important factors. To obtain a microcapsule of high density, there is known an art in that a microcapsule is prepared in the presence of PVA (PVA is used as a protective colloid or an emulsifying and dispersing agent). There has been observed a certain correlation between the density of the wall of a microcapsule and the saponification degree of PVA. Thus, as the protective colloid, PVA having a saponification degree of not lower than 95% is employed. The expected effect, that is, formation of microcapsule wall with high density cannot be attained in the case that PVA having a saponification degree of not higher than 90% is employed.

The present inventor has discovered that a reagent for immunological determination employing a microcapsule as the carrier shows high sensitivity or S/N ratio in the case that PVA having a saponification degree of not higher than 90% is provided to the surface of the microcapsule. It has been also discovered that the same effect can be attained in the use of other particulate carriers. Further, the same effect can be attained even in the use of a sheet-type carrier or any other type of carrier which is generally used in an immunological determination utilizing an emzyme-labeled immunologically active component.

The saponification degree of PVA employed in the present invention for the provision to the surface of a carrier should not exceed 90%. PVA having a higher saponification degree than 90%, for example, PVA whose saponification degree is 95% generally employed for the preparation of microcapsules for pressure sensitive paper cannot bring about the effect provided by the use of PVA defined in the present invention. The upper limit of the saponification degree of PVA for attaining the desired effect is very critical. The lower limit thereof is not so critical as the upper limit. The lowest saponification degree is supposed to be approx. 70%, taking into consideration availability, stability and reproducibility of such PVA. PVA having a saponification degree lower than 70% is generally _per_ _se_ colored, and thus is not suitable for a reagent for immunological determination.

The polymerization degree of PVA having saponification degree of not higher than 90% employed in the invention preferably ranges from 100 to 4000. PVA having a relatively lower polymerization degree, for example, not higher than 1000, is preferred for the reagent in the form of microcapsule or other microparticle, while PVA having a rather higher polymerization degree, for example, not lower than 1500 is suitable for the reagent for enzyme immunological determination.

The PVA having a saponification degree of not higher than 90% may be incorporated at any stage of the preparation of the reagent, as far as the PVA is provided to the surface of a final product. However, the PVA is preferably added at the following stage. The term "microcapsule reagent" employed in this specification means a reagent utilizing a microcapsule employable in microtiter method as a carrier.

For the preparation of the microcapsule reagent, the PVA having a saponification degree of not higher than 90% can be provided to the microcapsule at the following stage:

(1) Stage of addition of a protective colloid or an emulsifying and dispersing agent [e.g. poly(vinyl alcohol) having a saponification degree of not lower than 90% (e.g. PVA having a saponification degree of 98% and a polymerization degree of 500) in combination with poly-(styrenesulfonic acid)] in the emulsifying process: at this stage, the PVA having a saponification degree of not higher than 90% is employed in an amount of approx. 1 - 25% ("%" means "weight percent", unless otherwise indicated, this being applied to the following) of the PVA used as the protective colloid, etc..

(2) Stage after emulsifying procedure: the PVA having a saponification degree of not higher than 90% is employed in an amount of approx. 5 - 25% of the PVA used as the protective colloid, etc.

(3) Stage for removing unreacted residue of encapsulating reaction by washing with water: the PVA having a saponification degree of not higher than 90% is provided to the microcapsule using an aqueous solution containing 0.01 - 5% of the PVA.

(4) Stage after sensitization: the PVA having a saponification degree of not higher than 90% is provided to the microcapsule using an aqueous solution containing 0.01 - 5% of the PVA.

0135129

(5)  Stage for redispersing microcapsule reagent: the PVA having a saponification degree of not higher than 90% is provided to the microcapsule in the form of an aqueous solution (for redispersion) containing 0.01 - 5% of the PVA.

For the preparation of the reagent such as one in the form of a sheet (or strip) utilizing an enzyme-labeled immunologically active component, the PVA having a saponification degree of not higher than 90% can be provided to the sheet, etc. at the stage after the sensitization by immersing the sheet, etc., in an aqueous solution containing 0.01 - 5% of the PVA.

The reagent for immunological determination prepared by treating the carrier with PVA, a saponification degree of which is not higher than 90% shows the following effects.  The microcapsule reagent gives clearly and widely spread agglutination patterns with improved detection sensitivity.  Further, the sensitized solution is improved in the preservability.  The reagent for enzyme immunological determination decreases as for the blank level and accordingly increases in the sensitivity.

It is noted that in the case of a microcapsule reagent, a sensitized solution prepared by employing the PVA defined in the invention together with a protective colloid employing the combination of PVA having a saponification degree not lower than 90%, preferably not lower than 95% (e.g., PVA having a saponification degree of 98% and a polymerization degree of 500) and poly-(styrenesulfonic acid) is prominently improved in the preservability.

In the case of the reagent for enzyme immunological determination utilizing an enzyme-labeled immunologically active component, examples of the carrier in the form of a sheet including strip, test piece, etc. which shows a prominent effect of the PVA treatment employing PVA having a saponification degree of not higher than 90%,

- 7 -

0135129

include Teflon, cellulose triacetate, cellulose diacetate, polyethylene terephthalate, poly(methyl methacrylate), polysulfone, ABS resin, polycarbonate, polystyrene, poly(vinyl cholride), polyethylene and nylon.

The microcapsule employable as the carrier in the present invention consists essentially of a core of an oily material and a wall. A conventional process for the preparation of a microcapsule is described in detail in, for example, "Microcapsule" by Asashi Kondo (Nikkan Kogyo Shimbun Co Ltd., 1970). Detailed description on examples of the oily material, wall material and various kinds of additives is given, for instance, in Japanese Patent Provisional Publication Nos. 57(1982)-196621 and 57(1982)-196622.

The microcapsule can be sensitized with an antigen or antibody by causing the wall of the microcapsule to absorb an antigen or antibody in accordance with known methods.

The microcapsule used as the carrier preferably has a specific gravity ranging from 0.85 to 1.25 $g/cm^3$, and an mean particle size ranging from 0.5 to 20 μm, more preferably from 1 to 10 μm. The solid content desirably is approx. 1 - 3%.

The present invention will be further illustrated by the following examples.

## Example 1

(1) Preparation of Microcapsule

In a mixture (specific gravity: 1.14 $g/cm^3$) of 8.4 g. of diisopropyl naphthalene and 16.6 g. of chlorinated paraffin (Toyoparrux 150, manufactured by Toyo Soda Mfg. Co., Ltd., Japan, chlorination degree: 50%) was dissolved 0.25 g. of oil-soluble red fluorescent dye (Oleosol Red BB, manufactured by Sumitomo Chemical Co., Ltd., Japan, C.I. 26105). The resulting solution was mixed with 1.6

0135129

g. of 1,1,1-tris[(3-isocyanato-4-methylphenyl)carbamoyl-oxymethyl]propane (Vernock D-750, manufactured by Dai-nippon Ink & Chemicals Inc., Japan) dissolved in 2 g. of methyl ethyl ketone. Thus obtained oily liquid was added under stirring to an aqueous solution of 2 g. of poly-(vinyl alcohol) (PVA-105, manufactured by Kuraray Co., Ltd., Japan, saponification degree: 98%, polymerization degree: 500) and 1 g. of partial sodium salt of poly(styrenesulfonic acid) (manufactured by Proctor & Gamble Co., VERSA, TL-500, mean molecular weight: 500,000) in 75 ml. of water, and the resulting mixture was emulsified to obtain an emulsion having oily drops (mean size: approx. 5 μm.) To the resulting emulsion was added 2.6 g. of hexamethylenediamine dissolved in 25 ml. of water. After the addition was complete, the emulsion was diluted with 75 ml. of water and then a reaction was performed at 70°C for 2 hours to complete microencapsulation. Thus produced microcapsules were centrifugally washed with physiological saline solution to remove the unreacted residue, and dispersed in physiological saline solution so as to make a microcapsule dispersion having a microcapsule particle concentration of 10%.

(2) Preparation of Microcapsule Reagent

1.5 g. of the microcapsules prepared in the above (1) was diluted by dispersing them in 8.5 ml. of physio-logical saline solution. 2 ml. of a commercially avail-able anti-human albumin antibody (available from Kappel Corp., goat serum, IgG fraction) diluted with physiological saline solution as much as 40 times was introduced into 2 ml. of the microcapsule-containing diluent, and incubated at 37°C fo 90 minutes. After it was subjected to centrifugal separation, the precipitate was dispersed in 4 ml. of 0.15M phosphate-buffered physiological saline solution (PBS) containing 0.1% of poly(vinyl alcohol) having a saponification degree of not higher than 90%

- 9 -

0135129

(PVA-205, manufactured by Kuraray Co., Ltd., saponification degree: 88%, polymerization degree: 500), and incubated at 37$^{\circ}$C for 45 minutes. It was then subjected to centrifugally washing with PBS three times, and the mixture was dispersed in 2 mℓ. of PBS containing 3% bovine serum albumin (BSA) to prepare a reagent for detecting human albumin.

Comparison reagent A was prepared in the same manner as in Example 1-(2) except that the treatment with the PVA having a saponification degree of not higher than 90% was not carried out.

(3)  Evaluation of Microcapsule Reagent on Antibody Value

The reagent prepared in Example 1-(2) was subjected to an antigen-antibody reaction by the following procedures in accordance with microtiter method. The tube clearly showing aggulutination was judged to be positive. The maximum dilution number (multiple number) for the positive serum was obtained, and the obtained number was marked as antibody value.

1% human albumin (prepared by Kappel Corp.) solution was diluted as much as 10,000 times with PBS containing 1% BSA, and normal goat serum was diluted as much as 10 times with PBS containing 1% BSA as negative control. 25 µℓ of each was placed in holes of the microplates having V-shaped bottom, respectively. These portions were diluted with PBS containing 1% BSA so that a series of sample portions whose dilution ratio were sequentially increased by twice were prepared, that is, a lineup of multiple dilutions were prepared.

25 µℓ of the microcapsule reagent prepared in Example 1-(2) was taken in a dropper and dropwise applied to the holes of the dilution lineup of the sample liquid on the microplate. The microplate was shaken for 5 min. to accelerate the antigen-antibody reaction. After the microplate was allowed to stand at room temperature for 3 hours, agglutination patterns on the bottom of the microplate were observed and the antibody values were determined as shown in Table 1.

Comparison reagent A was processed in the same manner as described in the above (3) to determine the antibody-values as shown in Table 1.

Table 1

| Microcapsule Reagent | Antibody Value | |
| --- | --- | --- |
| | Human Albumin | Negative Control |
| Reagent of the Invention | 2,560,000 | 20 |
| Comparison Reagent A | 1,280,000 | 20 |

It was noted that when the reagent of the invention prepared by fixing the anti-human albumin antibody to the microcapsules and treating them with PVA having a saponification degree of not higher than 90% was employed in the microtiter method, clear and widely spreading agglutination patterns were formed. Thus obtained agglutination patterns were extremely easy to judge and the detection sensitivity thereof was higher than that obtained by the use of the control reagent A. On the other hand, agglutination patterns formed by the use of the control reagent A were notched in the circumference. Further, it was difficult to distinguish agglutination pattern from sediment because of the insufficient spreading of the pattern.

(4) Test for Preservability

The reagent of the invention and control reagent A prepared in Example 1-(2) were placed in vial bottles,

- 11 -

0135129

respectively. The bottles were sealed hermetically and stored in a refrigerator kept at 4°C for one month. Subsequently, the microtiter test was carried out in the same manner as described in the above (3) to determine antibody values as shown in Table 2.

Table 2  (after one month lapse)

| Microcapsule Reagent | Antibody Value | |
| --- | --- | --- |
| | Human Albumin | Negative Control |
| Reagent of the Invention | 640,000 | 10 |
| Comparison Reagent A | 160,000 | 10 |

The results set forth in Table 2 indicate that the deterioration in the sensitiveity of the reagent of the present invention after storage in the refrigerator is less than that of the control reagent A prepared without thetreatment by the PVA having saponification degree of not higher than 90%.

Example 2

Microcapsules were prepared in the same manner as in Example 1-(1) except that 0.2 g. of poly(vinyl alcohol) (PVA-205) was further added in 75 ml. of water in addition to 2 g. of PVA-105 and 1 g. of partial sodium salt of poly(styrenesulfonic acid) as high molecular protective colloid solution for emulsifying the oily liquid.

The anti-human albumin antibody was fixed to thus obtained microcapsules to prepare a reagent for detecting human albumin in the same manner as in Example 1-(2) except that PVA-205 treatment was not carried out.

0135129

## Example 3

Microencapsulation was carried out according to Example 1-(1). However, the microcapsules were centrifugally washed with simple physiological saline solution for the first time, physiological saline solution containing 0.1% poly(vinyl alcohol) having a saponification degree of not higher than 90% (PVA-205, saponification degree: 88%, polymerization degree: 500) for the second time, and simple physiological saline solution for the third time. The presipitate was dispersed in simple physiological saline solution so as to prepare a microcapsule dispersion having a microcapsule particle concentration of 10%.

The anti-human albumin antibody was fixed to the microcapsules in the same manner as in Example 1-(2) to prepare a reagent for detecting human albumin.

## Example 4

The anti-human albumin antibody was fixed to the microcapsule prepared in Example 1-(1) in the same manner as in Example 1-(2). The microcapsule was then centrifugally washed with PBS and dispersed in 2 ml. of PBS containing 2.5% BSA and 0.1% of PVA-205 to prepare a reagent for detecting human albumin.

## Evaluation of Microcapsule Reagent

Microcapsule reagents prepared in Examples 2 - 4 were evaluated in the same manner as in Example 1-(3) by the microtiter method. The similar test was carried out after storage of the reagents in a refrigerator kept at 4°C for one month  The results are set forth in Table 3.

Table 3

| Microcapsule | Antibody Value | | | |
|---|---|---|---|---|
| Reagent | Just after Preparation | | After 1 month | |
| | Human Albumin | Negative Control | Human Albumin | Negative Control |
| Example 2 | 5,120,000 | 80 | 1,280,000 | 20 |
| Example 3 | 2,560,000 | 20 | 640,000 | 10 |
| Example 4 | 2,560,000 | 40 | 1,280,000 | 20 |

The same effects as in Example 1, that is, improvement of agglutination patterns, increase in detection sensitivity and decrease in deterioration of the sensitivity during storage were observed by the incorporation of poly(vinyl alcohol) having a saponification degree of 88% at the stage of forming the microcapsule particles, at the stage of removing unreacted residue after formation of the microcapsule, or in the final reagent solution.

## Example 5

(1)  Preparation of Reagent for Enzyme Immunological Determination

A cellulose triacetate film (TAC film) was saponified by immersing in aqueous 2N sodium hydroxide solution at room temperature for 1 hour.  After being washed with water, the saponified product was cut into pieces of 1 cm square.  To 200 ml. of water was added 0.74 g. of cyanuric chloride in 3 ml. of dioxane.  100 pieces of the film cut into 1 cm. square were placed in the mixture.  The mixture was buffered to pH 7 under ice-cooling, made to react for 2 hours, and washed. 100 pieces of the divided film treated with cyanuric chloride were placed in an aqueous solution of 1.2 g. of hexamethylenediamine in 200 mℓ. of water, and a reaction was performed at 50$^{\circ}$C for 45 min. and further at 80$^{\circ}$C for 15 min. After removing unreacted residue by washing, 30 pieces out of the above film pieces were placed in 30 mℓ. of a solution prepared by diluting 25% glutaraldehyde as much as 100 times with physiological saline solution, and a reaction was performed at 37$^{\circ}$C for 60 min. After being washed, the above 30 film pieces were placed in 30 mℓ. of a solution prepared by diluting an anti-human IgG antibody (available from Miles Laboratories Inc.) as much as 50 times with physiological saline solution, and a reaction was performed at 37$^{\circ}$C for 3 hours. After being washed with physiological saline solution, 10 film pieces were immersed in each of the following solution for 30 min.

(i) PBS containing 1% PVA-217 (saponification degree: 88%, polymerization degree: 1700, manufactured by Kuraray Co., Ltd., Japan)

(ii) PBS containing 1% PVA-117 (saponification degree: 98%, polymerization degree: 1700, manufactured by Kuraray Co., Ltd.)

(iii) PBS

Subsequently, these film pieces were immersed in PBS containing 1% BSA to prepare a film for detecting human IgG.

(2) Evaluation of Reagent

Three kinds of the films (i), (ii) and (iii) prepared in Example 5-(1) were compared with one another in the blank level. The film of Example 5-(1), one piece for each, was immersed in 1 mℓ. of a solution prepared by diluting 1 % solution of goat IgG (available from Miles Laboratories Inc.) as much as 100 times with PBS, and incubated at 37$^{\circ}$C for 2 hours. After being washed with PBS, a piece of the film was immersed in 1 ml. of a solution prepared by diluting POD-labeled anti-human IgG (available from Miles Laboratories Inc., goat immunity) as much as 200 times with PBS containing 0.1% BSA, and incubated at 37°C for 90 min.  After being washed with PBS containing 0.1% BSA, the film was immersed in PBS containing 1% BSA and subjected to enzyme color formation test.

In 30 mℓ. of a solution buffered to pH 5 with sodium phosphate-citric acid were dissolved 12 mg. of o-phenylenediamine and 0.2 mℓ. of 0.5% hydrogen peroxide solution.  Into 2 mℓ. of the resulting solution kept at 37°C, a piece of the film subjected to the above treatment was placed.  After the solution was subjected to reaction at 37°C for 10 min., 3 mℓ. of 1-N sulfuric acid was added to the solution to complete the reaction.  The optical density of the reaction liquid at a wavelength of 492 nm was measured using 1 cm. cell by means of a spectrophotometer 200 type (manufactured by Hitachi Ltd.).  The results are set forth in Table 4.

Table 4

| Film | Optical Density (492 nm) |
|------|--------------------------|
| (i) according to the invention | 0.06 |
| (ii) for comparison | 0.43 |
| (iii) for comparison | 0.47 |

Accordingly, it was confirmed that the reagent treated with poly(vinyl alcohol) having a saponification degree of 88% according to the present invention is pro-

0135129

minently effective to lower the blank level of an enzyme
immunological reaction employing the sandwich method.

**0135129**

CLAIMS:

1. A reagent for immunological determination in the form of a carrier which is provided on the surface with poly(vinyl alcohol) having a saponification degree of not higher than 90% and sensitized with an immunologically active component.

2. The reagent for immunological determination as claimed in claim 1, wherein said poly(vinyl alcohol) has saponification degree ranging from 70 - 90%.

3. The reagent for immunological determination as claimed in claim 1 or claim 2 wherein said immunologically active component is an antigen or antibody and said carrier is a microcapsule.

4. The reagent for immunological determination as claimed in claim 1 wherein said carrier is sensitized with an enzyme-labeled immunologically active component.